# EUROPÄISCHE PATENTSCHRIFT

(11) **EP 2 112 936 B1**
(45) Veröffentlichungstag und Bekanntmachung des Hinweises auf die Patenterteilung: **14.12.2011**
(21) Anmeldenummer: 08707017.3
(22) Anmeldetag: 12.01.2008
(51) Int. Cl.: A61M 1/02

(54) **VORRICHTUNG UND VERFAHREN ZUM FILTRIEREN EINER MISCHUNG EINER BLUTKOMPONENTE UND EINER ADDITIVLÖSUNG**
APPARATUS AND METHOD FOR FILTERING A MIXTURE OF A BLOOD COMPONENT AND AN ADDITIVE SOLUTION
DISPOSITIF ET PROCÉDÉ DE FILTRATION D'UN MÉLANGE COMPOSÉ D'UN CONSTITUANT SANGUIN ET D'UNE SOLUTION ADDITIVE

(30) Priorität: 16.01.2007 DE 102007002291
(43) Veröffentlichungstag der Anmeldung: 04.11.2009
(73) Patentinhaber: Fresenius HemoCare Deutschland GmbH, 61352 Bad Homburg (DE)
(72) Erfinder: MEISBERGER, Arthur, 66606 St. Wendel (DE); NEUMANN, Hans-Jürgen, 66606 St. Wendel (DE)
(74) Vertreter: Luderschmidt, Schüler & Partner
(86) Internationale Anmeldenummer: PCT/EP2008/000212
(87) Internationale Veröffentlichungsnummer: WO 2008/086988

(56) Entgegenhaltungen:
- WO-A-95/00631
- WO-A-03/055581
- GB-A- 2 142 835
- US-A- 3 460 809
- US-A- 5 269 946
- US-A- 5 836 934
- US-A1- 2004 256 329
- US-A1- 2006 051 448
- US-A1- 2006 226 057

## Beschreibung

Die Erfindung bezieht sich auf eine Vorrichtung zum Filtrieren einer Mischung aus einer Blutkomponente und einer Additivlösung für die Blutkomponente. Darüber hinaus betrifft die Erfindung ein Verfahren, bei dem eine Blutkomponente und eine Additivlösung für die Blutkomponente miteinander gemischt und die Mischung aus Blutkomponente und Additivlösung gefiltert werden.

Es sind verschiedene Vorrichtungen und Verfahren zur Gewinnung von aus bestimmten Blutkomponenten bestehenden Konzentraten bekannt. Zur Gewinnung einer Blutkomponente kann das Blut eines Spenders einer Zentrifugation unterworfen werden, wodurch das Blut in verschiedene Blutkomponenten getrennt wird.

Die gewonnenen Blutkomponenten werden im Allgemeinen mit einer Additivlösung gemischt, wobei die Mischung aus Blutkomponente und Additivlösung filtriert wird, um unerwünschte Bestandteile zu entfernen.

Während die Gewinnung der Blutkomponenten mit den bekannten Blutverarbeitungsvorrichtungen im Wesentlichen vollautomatisch erfolgt, können die gewonnenen Blutkomponenten mit den bekannten Blutverarbeitungsvorrichtungen ohne manuelle Eingriffe nicht filtriert werden.

Zum Filtrieren einer Blutkomponente ist es erforderlich, die Blutkomponente mit einer Additivlösung zu mischen. Beispielsweise wird ein Konzentrat aus Erythrozyten RCC, das in einem Konzentratbeutel bereitgestellt wird, mit einer Lagerlösung (SAG-M) gemischt, die sich in einem Vorratsbeutel befindet. Nach dem Mischen von Erythrozytenkonzentrat und Lagerlösung ist eine schonende und schnelle Filtration des Blutproduktes möglich, das in einem Sammelbeutel gelagert wird.

Die Filtration des Blutproduktes mit den bekannten Filtern erfordert eine bestimmte Viskosität des Blutproduktes, beispielsweise des Erythrozytenkonzentrates, die durch das Mischungsverhältnis von Blutkomponente und Additivlösung eingestellt wird. Weiterhin darf in Abhängigkeit von den verwendeten Filtern eine vorgegebene Durchflussmenge durch den Filter und ein vorgegebener Innendruck im Filter nicht überschritten werden.

Die US-A-5,836,934 beschreibt eine Vorrichtung zum Mischen einer Blutkomponente mit einer Additivlösung. Die mit der Additivlösung zu vermischende Blutkomponente befindet sich zusammen mit anderen Blutkomponenten in einem ersten Beutel, während sich die Additivlösung in einem zweiten Beutel befindet. In dem ersten Beutel sind die Blutkomponenten schichtweise angeordnet, wobei das Erythrozytenkonzentrat die unterste Schicht bildet.

Zum Abtrennen des Erythrozytenkonzentrats und zum Vermischen des Erythrozytenkonzentrats mit der Additivlösung werden beide Beutel zwischen zwei Anpressplatten angeordnet, die relativ zueinander bewegt werden, so dass die Beutel zusammengedrückt werden.

Von dem unteren Ende des Konzentratbeutels und des Additivlösungsbeutels geht jeweils eine Schlauchleitung ab, die zu einem Y-Verbindungsstück führen, von dem eine Schlauchleitung abgeht, an der ein Filter angeschlossen ist. Erythrozytenkonzentrat und Additivlösung, die in dem Y-Verbindungsstück zusammengeführt werden, sollen sich in der zu dem Filter führenden Schlauchleitung vermischen. Nachteilig ist, dass eine optimale Vermischung von Erythrozytenkonzentrat und Additivlösung in der Schlauchleitung nicht sichergestellt ist. Für den Fall, dass die Viskosität der Mischung aber nicht optimal eingestellt ist, besteht die Gefahr, dass sich der Filter zusetzt. Weiterhin verlängert sich bei nicht optimal eingestellter Viskosität die Prozesszeit.

Die aus der US-A-5,836,934 bekannte Vorrichtung verfügt über eine Regelungsvorrichtung für die Anpressplatten, die derart ausgebildet ist, dass die einzelnen Blutkomponenten aus dem Blutbeutel abgezogen werden. Die Regelung verhindert beispielsweise, dass das Erythrozytenkonzentrat in die Schlauleitung strömt, die von der Oberseite des Blutbeutels abgeht.

Aus der EP-A-1 052 898 ist eine Vorrichtung zur Aufbereitung einer Blutkomponente zur Kryokonservierung durch Vermischen der Blutkomponente mit einer Kryokonservierungslösung bekannt, bei der zur Verringerung des Risikos eines Osmolaritätsschocks der Blutkomponente entweder ein bestimmtes Volumen einer Kryokonservierungslösung oder einer Verdünnungslösung zugeführt wird. Die Förderung der beiden Lösungen erfolgt mit Pumpen, deren Förderraten von einem Regler eingestellt werden.

Die US 2006/0226057 A1 beschreibt eine Vorrichtung zum Filtrieren einer Blutkomponente und einer Additivlösung, die eine erste Kammer zur Aufnahme einer Blutkomponente und eine zweite Kammer zur Aufnahme einer Additivlösung für die Blutkomponente umfasst. Die bekannte Vorrichtung verfügt über Mittel zum Mischen der Blutkomponente und der Additivlösung, wobei die Blutkomponente über eine Leitung und die Additivlösung über eine Leitung den Mitteln zum Mischen der Blutkomponente und der Additivlösung zugeführt wird. Eine weitere Leitung verbindet die Mittel Mischen der Blutkomponente und der Additivlösung mit Mitteln zum Filtrieren der Mischung von Blutkomponente und Additivlösung.

Die Mittel zum Mischen der Blutkomponente und der Additivlösung werden als statischer Mischer bezeichnet, der aber nicht näher beschrieben wird.

Der Erfindung liegt die Aufgabe zugrunde, eine Vorrichtung zum Filtrieren einer Mischung aus einer Blutkomponente und einer Additivlösung bereitzustellen, mit der eine schnelle und schonende Filtration des Blutproduktes durchgeführt werden kann. Darüber hinaus ist eine Aufgabe der Erfindung, ein Verfahren anzugeben, dass eine schonende und schnelle Filtration der Mischung aus einer Blutkomponente und einer Additivlösung erlaubt.

Die Lösung dieser Aufgaben erfolgt erfindungsgemäß mit den Merkmalen der **unabhängigen** Patentansprüche Vorteilhafte Ausführungsformen der Erfindung sind Gegenstand der Unteransprüche.

Bei der erfindungsgemäßen Vorrichtung zum Filtrieren einer Blutkomponente und einer Additivlösung verfügen die Mittel zum Mischen der Blutkomponente und der Additivlösung Verwirbelungen erzeugende Verwirbelungselemente. Durch die Verwirbelungen in der Flüssigkeitsströmung wird erreicht, dass die Blutkomponente und die Additivlösung optimal vermischt werden, so dass sich die Mischung aus Blutkomponente und Additivlösung schonend und schnell filtrieren lässt.

Die Erfindung sieht vor, dass die Mittel zum Mischen der Blutkomponente und der Additivlösung einen von der Blutkomponente und der Additivlösung durchströmten Körper aufweisen, dessen Strömungsquerschnitt zur Ausbildung der Verwirbelungselemente in vorgegebenen Abschnitten mit unterschiedlichem Strömungsquerschnitt ausgebildet ist. Die wechselnden Strömungsquerschnitte begünstigen das Vermischen der Flüssigkeiten. Dabei kommt es im Wesentlichen darauf an, dass der Strömungsquerschnitt seine Form, nicht aber seine Größe ändert. Es ist aber auch möglich, dass mit der Veränderung der Form des Strömungsquerschnitts auch eine Veränderung der Größe des Strömungsquerschnitts verbunden ist.

Die Erfindung sieht vor, dass die erste und zweite Flüssigkeitsleitung, die von der ersten und zweiten Kammer zu den Mitteln zum Mischen der Blutkomponente und der Additivlösung führen, zu einer gemeinsamen Flüssigkeitsleitung führen, wobei der von der Blutkomponente und der Additivlösung durchströmte Körper Teil der gemeinsamen Flüssigkeitsleitung ist. Die gemeinsame Flüssigkeitsleitung ist eine Schlauchleitung, die unter Ausbildung der Verwirbelungselemente in einem vorgegebenen Abschnitt der Schlauchleitung verformt ist. Damit können die Mittel zum Mischen der Blutkomponente und der Additivlösung Teil eines zur einmaligen Verwendung bestimmten Schlauchsets (Disposible) sein, das sich in großen Stückzahlen einfach und kostengünstig herstellen lässt.

Die Mittel zum Mischen der Blutkomponente und der Additivlösung weisen eine Vorrichtung zum Einlegen der Schlauchleitung auf, wobei die Vorrichtung an der Schlauchleitung angreifende Verformungselemente aufweist, mit denen die Schlauchleitung derart verformt wird, dass sich die Verwirbelungselemente ausbilden. Die Vorrichtung zum Einlegen der Schlauchleitung kann beispielsweise als Clip ausgebildet sein, in den die Schlauchleitung eingelegt wird.

Bei einer weiteren besonders bevorzugten Ausführungsform ist der Strömungsquerschnitt des von der Blutkomponente und der Additivlösung durchströmten Körpers in vorgegebenen Abständen derart ausgebildet, dass der Abstand zwischen zwei auf einer ersten Achse gegenüberliegenden Punkten des Körpers geringer als der Abstand zwischen zwei gegenüberliegenden Punkten ist, die auf einer zweiten im rechten Winkel zu der ersten Achse verlaufenden Achse liegen. Vorzugsweise weist der von der Blutkomponente und der Additivlösung durchströmte Körper in vorgegebenen Abständen ein Strömungsquerschnitt in Form einer im Wesentlichen ovalen Fläche, insbesondere einer Ellipse auf, wobei die Hauptachsen der in vorgegebenen Abständen aufeinanderfolgenden Strömungsquerschnitte in unterschiedliche Richtungen verlaufen. Der ständige Wechsel der Form des Strömungsquerschnitts führt zu einer schnellen Durchmischung der Blutkomponente und der Additivlösung, wobei sich die Viskosität der Mischung optimal einstellen lässt.

Die bevorzugten Ausführungsformen der erfinderischen Mischvorrichtung sind von eigener erfinderischer Bedeutung, d.h. die Mischvorrichtung kann grundsätzlich auch in einem anderen Zusammenhang als in dem vorliegenden Schlauchset Verwendung finden. Des weiteren ist es möglich, eine Mischvorrichtung als Bestandteil des Filters des vorliegenden Schlauchsets vorzusehen. So kann die Mischvorrichtung als Einlassstück des Filters ausgebildet sein oder auch Teil des Filtervlieses oder der Membran sein.

Das erfindungsgemäße Verfahren zum Filtrieren von Blut sieht vor, dass die Blutkomponente und die Additivlösung vor dem Filtrieren verwirbelt werden, so dass Blutkomponente und Additivlösung optimal vermischt werden und die Mischung von Blutkomponente und Additivlösung die erforderliche Viskosität hat, bei der die Filtration in möglichst kurzer Prozesszeit durchgeführt werden kann.

Im Folgenden werden Ausführungsbeispiele der Erfindung unter Bezugnahme auf die Zeichnungen näher erläutert.

Es zeigen:
- Figur 1: Ein erstes Ausführungsbeispiel der erfindungsgemäßen Vorrichtung zum Filtrieren von Blut in stark vereinfachter schematischer Darstellung,
- Figur 2: den Ausschnitt A von Figur 1 in vergrößerter Darstellung,
- Figur 3: einen Schnitt entlang der Linie III-III von Figur 2 in vergrößerter Darstellung,
- Figur 4: einen Schnitt entlang der Linie IV-IV von Figur 2 in vergrößerter Darstellung,
- Figur 5: einen Schnitt entlang der Linie V-V von Figur 2 in vergrößerter Darstellung,
- Figur 6: eine alternative Ausführungsform der Mittel zum Mischen von Blutkomponente und Additivlösung in stark vereinfachter schematischer Darstellung, die aber nicht Gegenstand der Erfindung ist, und
- Figur 7: ein weiteres Ausführungsbeispiel der erfindungsgemäßen Filtriervorrichtung in stark vereinfachter schematischer Darstellung.

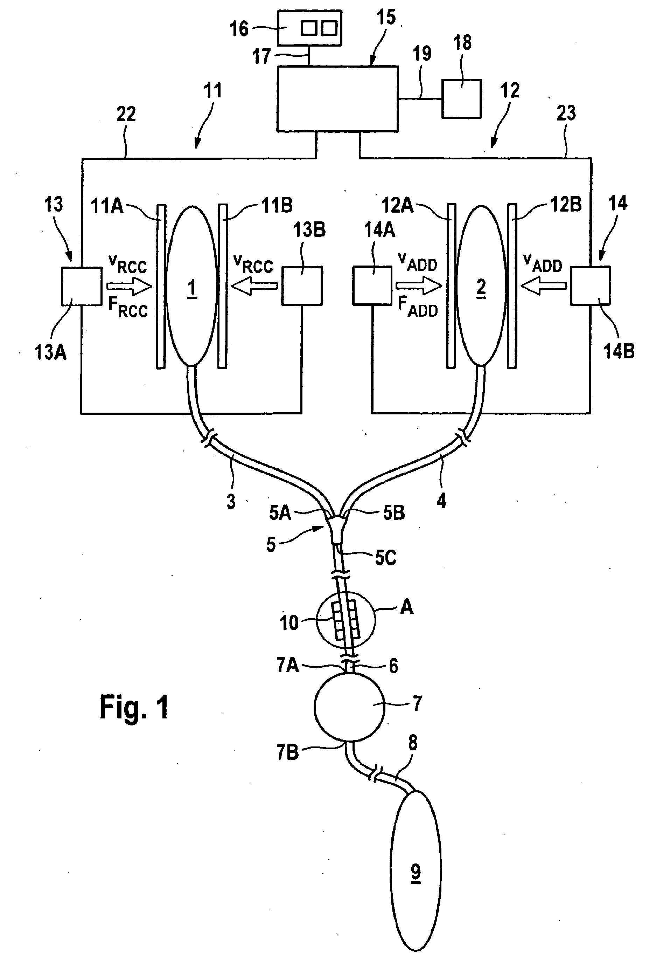

Figur 1 zeigt in stark vereinfachter schematischer Darstellung die wesentlichen Komponenten der erfindungsgemäßen Vorrichtung zum Filtrieren von einer Mischung aus einer Blutkomponente und einer Additivlösung. Die Blutkomponente, im vorliegenden Ausführungsbeispiel ein Erythrozytenkonzentrat (RCC), wird in einem ersten Beutel 1 und die Additivlösung, im vorliegenden Ausführungsbeispiel eine Lagerlösung (SAG-M), wird in einem zweiten Beutel 2 bereitgestellt. Bei den Beuteln handelt es sich um die bekannten Folienbeutel mit den bekannten Anschlusselementen. Von dem ersten Beutel 1 für das Erythrozytenkonzentrat geht eine erste Schlauchleitung 3 ab, während von dem zweiten Beutel für die Lagerlösung eine zweite Schlauchleitung 4 abgeht. Das Ende der ersten Schlauchleitung 3 ist an dem einen Einlass 5A und das Ende der zweiten Schlauchleitung 4 ist an dem anderen Einlass 5B eines Y-Verbindungsstücks 5 angeschlossen, während von dem Auslass 5C des Y-Verbindungsstücks 5 eine gemeinsame Schlauchleitung 6 abgeht, die zu dem Einlass 7A eines Filters 7 führt. Von dem Auslass 7B des Filters 7 geht eine weitere Schlauchleitung 8 ab, die zu einem Sammelbeutel 9 für die filtrierte Mischung von Erythrozytenkonzentrat und Lagerlösung führt. Der Erythrozytenbeutel 1, der Lagerlösungsbeutel 2 und der Sammelbeutel 9 bilden zusammen mit den Schlauchleitungen 3, 4, 6, 8 und dem Filter 7 ein zur einmaligen Verwendung bestimmtes Disposable.

Zwischen dem Y-Verbindungsstück 5 und dem Filter 7 zum Filtrieren der Mischung von Blutkomponente und Additivlösung befinden sich in der Schlauchleitung 6 Mittel 10 zum Mischen der Blutkomponente und der Additivlösung, die nachfolgend noch im Einzelnen beschrieben werden.

Die erfindungsgemäße Vorrichtung zum Filtrieren verfügt über eine erste Pressvorrichtung 11 zum Zusammendrücken des ersten Beutels 1, im vorliegenden Ausführungsbeispiel des Erythrozytenbeutels, und eine zweite Pressvorrichtung 12 zum Zusammendrücken des zweiten Beutels 2, im vorliegenden Ausführungsbeispiel des Lagerlösungsbeutels. Die erste und zweite Pressvorrichtung 11, 12 sind in Figur 1 nur andeutungsweise dargestellt. Die Pressvorrichtungen 11, 12 verfügen jeweils über zwei gegenüberliegende Andruckelemente 11A, 11B bzw. 12A, 12B, zwischen denen der Erythrozytenbeutel 1 und der Lagerlösungsbeutel 2 angeordnet sind.

Die Andruckelemente 11 A, 11 B der ersten Pressvorrichtung 11 und die Andruckelemente 12A, 12B der zweiten Pressvorrichtung 12 können jeweils relativ zueinander bewegt werden. Beim vorliegenden Ausführungsbeispiel werden jeweils beide Andruckelemente gleichzeitig aufeinander zu bewegt oder voneinander weg bewegt.

Zum Bewegen der Andruckelemente verfügt die erste und zweite Pressvorrichtung 11, 12 jeweils über eine Antriebsvorrichtung 13, 14, wobei jede Antriebsvorrichtung 13, 14 zwei Antriebseinheiten 13A, 13B bzw. 14A, 14B umfaßt, die jeweils an einem der Andruckelemente 11A, 11B bzw. 12A, 12B angreifen. Die Antriebsvorrichtungen sind in Figur 1 wieder nur schematisch angedeutet. Sie können unterschiedlich ausgebildet sein. Vorzugsweise können die Antriebsvorrichtungen elektromotorische oder pneumatische Antriebe sein.

Die Antriebsvorrichtungen 13, 14 bewegen die Anpresselemente mit einer vorgegebenen Geschwindigkeit V_{RCC} bzw. V_{ADD} unter Zwischenlage des Erythrozytenbeutels 1 und des Lagerlösungsbeutels 12 aufeinander zu und üben auf den Beutel 1 und den Beutel 2 eine vorgegebene Anpresskraft F_{RCC} bzw. F_{ADD} aus.

Darüber hinaus verfügt die erfindungsgemäße Vorrichtung zum Filtrieren über eine Steuervorrichtung 15, die über Steuerleitungen 22, 23 mit den Antriebseinheiten 13A, 13B bzw. 14A, 14B der Andruckelemente 13 bzw. 14 verbunden ist, und eine Eingabeeinheit 16, die mit der Steuervorrichtung 15 über eine Datenleitung 17 verbunden ist. Bei der Eingabeeinheit 16 kann es sich beispielsweise um eine Tastatur oder eine Einheit zum Einlesen von Daten handeln.

Mit der Eingabeeinheit 16 werden vorgegebene Grenzwerte vorgegeben. Die vorgegebenen Grenzwerte können aber auch bereits in einem Speicher abgelegt sein. Zu den vorgegebenen Grenzwerten zählen der Maximaldruck in dem Filter 7, die maximale Durchflussmenge durch den Filter 7, der maximale Innendruck in dem ersten und/oder zweiten Beutel 2 und ein vorgegebener Grenzwert für die Viskosität der Mischung von Blutkomponente und Additivlösung, die nicht unterschritten werden sollte, da ansonsten die Gefahr des Zusetzens des Filters besteht. Da die einzelnen Grenzwerte teilweise voneinander abhängig sind, ist es nicht zwingend erforderlich, für alle Größen Grenzwerte vorzugeben.

Die oben genannten Größen werden während des Filtrationsprozesses ständig überwacht. Hierzu verfügt die Filtrationsvorrichtung über eine Überwachungseinheit 18, die über eine Datenleitung 19 mit der Steuervorrichtung 15 verbunden ist.

Die einzelnen Größen können mit in Figur 1 nicht dargestellten Sensoren gemessen werden, die Bestandteil der Überwachungseinheit 18 sind. Die hierzu erforderlichen Sensoren, beispielsweise Drucksensoren oder Durchflusssensoren sind dem Fachmann bekannt. Es ist aber auch möglich einzelne Größen zu berechnen. Beispielsweise kann der Innendruck in dem Beutel 1 und 2 dadurch bestimmt werden, dass die von der Pressvorrichtung 1 bzw. 2 ausgeübte Anpresskraft F_{RCC} bzw. F_{ADD} ermittelt wird, und der Quotient von der Anpresskraft und der Anlagefläche des Beutels 1, 2 an dem jeweiligen Andruckelement berechnet wird. Hierzu verfügt die Steuervorrichtung 15 über eine entsprechende Recheneinheit.

Bei dem Prozess wird angestrebt, dass die Blutkomponente mit der Additivlösung in möglichst kurzer Prozesszeit filtriert wird, wobei die oben genannten Grenzwerte nicht überschritten bzw. unterschritten werden sollten. Insbesondere wird angestrebt, eine Viskosität für die Mischung von Blutkomponente und Additivlösung vorzugeben, bei der die Filtration in möglichst kurzer Zeit erfolgt. Dabei wird vorausgesetzt, dass sich zum Ende des Prozesses beide Beutel gleichzeitig entleert haben. Denn nur dann ist das richtige Mischungsverhältnis von Erythrozytenkonzentrat und Lagerlösung gegeben, das durch die Volumina der Beutel 1, 2 bzw. deren Füllvolumen bestimmt wird.

Nachfolgend wird die Funktionsweise der erfindungsgemäßen Filtrationsvorrichtung beschrieben.

Mit der Eingabeeinheit 16 werden die vorgegebenen Grenzwerte eingegeben, soweit die Grenzwerte nicht bereits in einer Speichereinheit abgelegt sind. Dann wird der Erythrozytenbeutel 1 und der Additivlösungsbeutel 2 zwischen die Andruckelemente 11A, 11B bzw. 12A, 12B der Pressvorrichtungen 11, 12 gelegt. Daraufhin wird die Vorrichtung in Betrieb gesetzt, wobei die Antriebsvorrichtungen 13, 14 die Andruckelemente 11A, 11B bzw. 12A, 12B aufeinander zu bewegen, so dass die Beutel 1, 2 ausgepresst werden. Die Steuervorrichtung 15 regelt die Geschwindigkeit V_{RCC} und V_{ADD}, mit denen die Andruckelemente von den Antriebsvorrichtungen bewegt werden, derart, dass die Grenzparameter des maximalen Innendrucks im Filter 7, der maximalen Durchflussmenge durch den Filter 7 sowie der maximale Innendruck in den Beuteln 1, 2 gerade erreicht, nicht aber überschritten werden. Dabei berücksichtigt die Steuervorrichtung 15 bei der Regelung, dass beide Beutel 1, 2 zum Ende des Mischprozesses gleichzeitig entleert sind. Die Bedingung des gleichzeitigen Entleerens beider Beutel hat bei der Regelung Priorität, da sichergestellt werden muss, dass das Mischungsverhältnis korrekt ist.

Der verbleibende Inhalt von Erythrozytenkonzentrat und Lagerlösung in den Beuteln 1 und 2 während des Filtrationsprozesses wird über das bekannte Füllvolumen und die Durchflussmenge durch die erste und zweite Schlauchleitung 3, 4 bestimmt, wobei das Füllvolumen mit der Eingabeeinheit 16 eingegeben wird oder bereits in dem Speicher abgelegt ist. Die Durchflussmengen werden mit der Überwachungseinrichtung 18 erfasst.

Der oben beschriebene Prozessablauf zur Erzielung einer möglichst kurzen Prozesszeit bei gleichzeitiger Entleerung beider Beutel ist von eigener erfinderischer Bedeutung.

Nachfolgend werden die Mittel 10 zum Mischen von Blutkomponente und Additivlösung im Einzelnen beschrieben, die ebenfalls von eigener erfinderischer Bedeutung sind. Beispielsweise ist es denkbar, die Mittel 10 zum Mischen, nicht aber die Steuerungsvorrichtung 15 mit der Überwachung der Parameter vorzusehen oder auch umgekehrt.

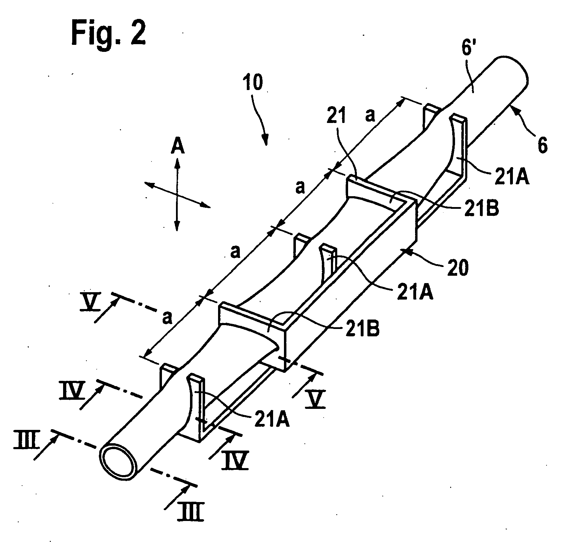

Die Figuren 2 bis 5 zeigen ein erstes Ausführungsbeispiel der Mittel 10 zum Mischen von Blutkomponente und Additivlösung. Bei dieser bevorzugten Ausführungsform, die bei dem Ausführungsbeispiel von Figur 1 verwirklicht ist, ist die gemeinsame Schlauchleitung 6, die von dem Y-Verbindungsstück 5 zu dem Filter 7 führt, in eine Einrichtung 20 eingelegt, bei der es sich um einen Kunststoff-Clip handelt. Dadurch wird ein rohrförmiger Körper mit einem kreisförmigen Strömungsquerschnitt geschaffen, der in vorgegebenen Abständen a verformt ist, so dass Verwirbelungen erzeugende Verwirbelungselemente entstehen.

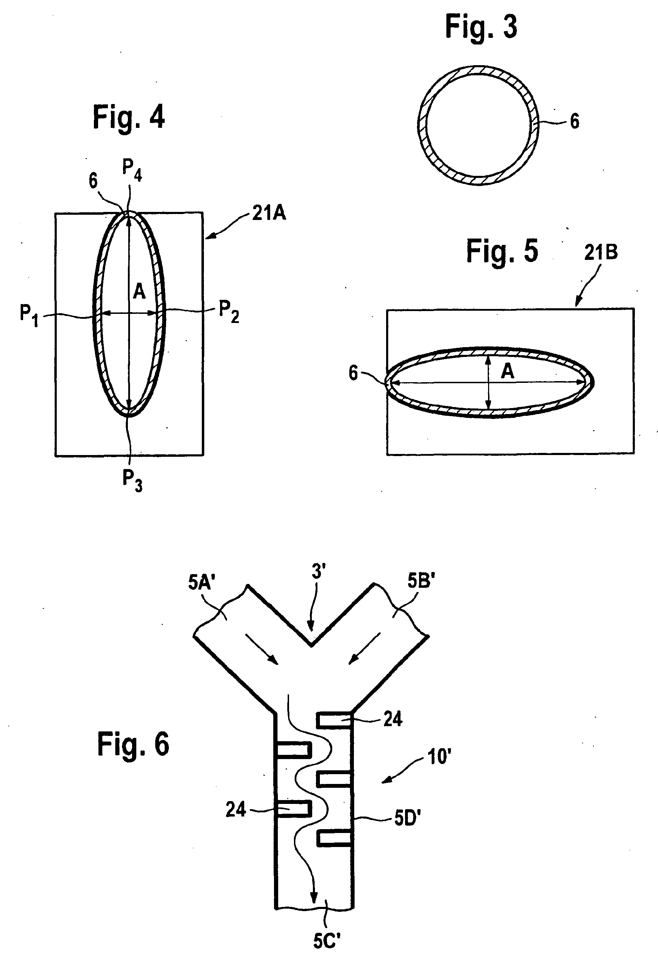

Figur 2 zeigt den in den Kunststoff-Clip 20 sitzenden Schlauchleitungsabschnitt 6' der Schlauchleitung 6. Die Schlauchleitung 6 hat den in Figur 3 gezeigten kreisförmigen Strömungsquerschnitt. Der Clip 20 verfügt über insgesamt 4 im Abstand a zueinander angeordnete Kunststoffstege 21, die Verformungselemente bilden, mit denen der Schlauch verformt wird. Der Clip 20 weist zwei Gruppen von Verformungselementen 21A, 21B auf, wobei beide Verformungselemente Kunststoffstege mit einer Ausnehmung sind, die einen ovalen, vorzugsweisen elliptischen Querschnitt haben. Figur 4 zeigt den Querschnitt des Stegs 21A, während Figur 5 den Querschnitt des Stegs 2 1 B zeigt. Die Hauptachsen A der elliptischen Ausnehmungen beider Stege 21A und 21B, in denen der Schlauch 6 verläuft, stehen senkrecht zueinander, wobei sich die Stege 21A und 21B jeweils in axialer Richtung einander abwechseln. Der in den Stegen liegende Schlauch 6 wird also in unterschiedliche Richtungen abwechselnd elliptisch verformt, wobei beim vorliegenden Ausführungsbeispiel die Hauptachsen A senkrecht aufeinander stehen.

Es hat sich gezeigt, dass mit einer derartigen Verformung des Schlauchs Verwirbelungen erzeugt werden, die das Durchmischen der Flüssigkeiten verbessern. Die Blutkomponente und die Additivlösung werden in dem kurzen Schlauchleitungsabschnitt 6' bei der relativ hohen Durchflussgeschwindigkeit, die für die kurze Prozesszeit erforderlich ist, ausreichend vermischt, so dass nicht die Gefahr des Zusetzens des Filters 7 besteht.

Bei der Verformung der Schlauchleitung 6 ist vorteilhaft, wenn jeweils zwei gegenüberliegende Punkte P₁, P₂ in einer Richtung einen kleineren bzw. größeren Abstand als zwei gegenüberliegende Punkte P₃, P₄ in einer anderen Richtung, vorzugsweise in einer dazu senkrecht stehenden Richtung haben.

Die Figuren 2 bis 5 zeigen ein Ausführungsbeispiel, bei dem die Verformungselemente Bestandteil eines Kunststoff-Clips sind. Die Verformungselemente können aber beispielsweise auch durch Andruckelemente oder Stößel einer extern Einrichtung gebildet werden, die von außen in bestimmten Abständen an der Schlauchleitung angreifen. Beispielsweise können die Andruckelemente Bestandteil einer Einheit der Filtrationsvorrichtung sein, in die die Schlauchleitung eingelegt wird.

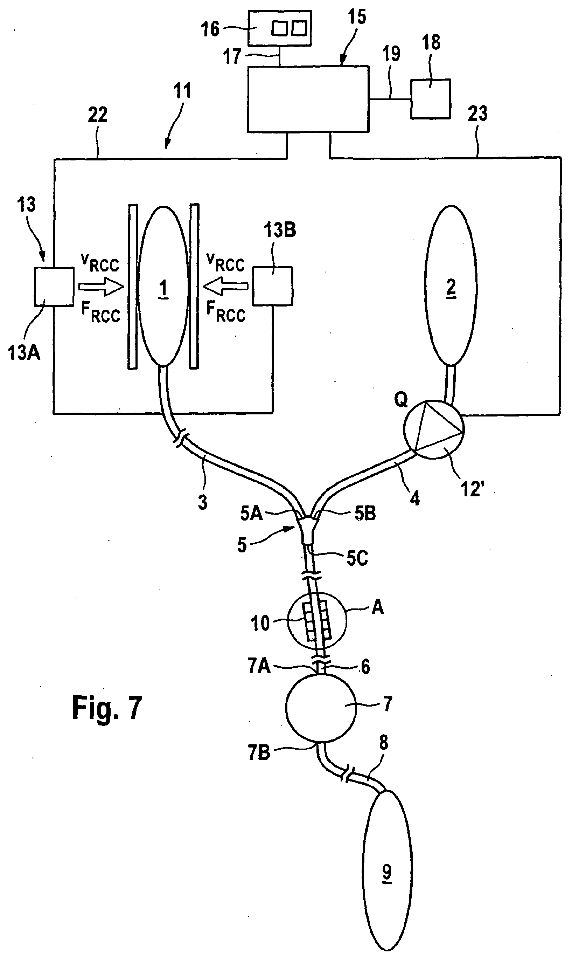

Figur 6 zeigt in stark vereinfachter schematischer Darstellung eine alternative Ausführungsform der Mittel 10' zum Mischen der Blutkomponente und der Additivlösung. Bei diesem Ausführungsbeispiel sind die Verwirbelungselemente Bestandteil des Y-Verbindungsstücks 5'. Die Verwirbelungselemente sind stromab des Ein- und Auslasses 5A', 5B' des Y-Verbindungsstücks 5' in dem gemeinsamen Schenkel 5D' vor dem Auslass 5C' des Verbindungsstücks angeordnet. Es sind abwechselnd von unterschiedlichen Seiten nach innen vorspringende Stege oder Vorsprünge 24, die zu Verwirbelungen führen. Figur 7 zeigt eine alternative Ausführungsform der erfindungsgemäßen Filtrationsvorrichtung, die sich von dem unter Bezugnahme auf Figur 1 beschriebenen Ausführungsbeispiel dadurch unterscheidet, dass anstelle der Anpressvorrichtung 12 für den Additivlösungsbeutel 1 eine Pumpe 12' vorgesehen ist, die in die erste von dem Additivlösungsbeutel 2 zu dem Filter 7 führende Leitung 4 geschaltet ist. Die einander entsprechenden Teile sind mit den gleichen Bezugszeichen versehen. Bei diesem Ausführungsbeispiel regelt die Steuervorrichtung 15 nicht die Andruckgeschwindigkeit V_{ADD} oder Anpresskraft F_{ADD} von den Andruckelementen einer Pressvorrichtung 12, sondern die Förderrate Q der Pumpe 12', die über die Steuerleitung 23 mit der Steuervorrichtung 15 verbunden ist.

## Patentansprüche

1. Vorrichtung zum Filtrieren einer Blutkomponente und einer Additivlösung mit
einer ersten Kammer (1) zur Aufnahme einer Blutkomponente,
einer zweiten Kammer (2) zur Aufnahme einer Additivlösung für die Blutkomponente,
Mitteln (10) zum Mischen der Blutkomponente und der Additivlösung, die einen von der Blutkomponente und der Additivlösung durchströmten Körper aufweisen, dessen Strömungsquerschnitt zur Ausbildung von Verwirbelungen erzeugenden Verwirbelungselementen in vorgegebenen Abständen (a) unterschiedlich ausgebildet ist,
einer ersten Flüssigkeitsleitung (3), die von der ersten Kammer zu den Mitteln zum Mischen der Blutkomponente und der Additivlösung führt und
einer zweiten Flüssigkeitsleitung (4), die von der zweiten Kammer zu den Mitteln zum Mischen der Blutkomponente und der Additivlösung führt, und
einer dritten Flüssigkeitsleitung (6), die von den Mitteln (10) zum Mischen der Blutkomponente und der Additivlösung zu Mitteln (7) zum Filtrieren der Mischung von Blutkomponente und Additivlösung führt,
wobei
die erste Flüssigkeitsleitung (3) und die zweite Flüssigkeitsleitung (4) zu einer gemeinsamen Flüssigkeitsleitung (6) führen, und der von der Blutkomponente und der Additivlösung durchströmte Körper Teil der gemeinsamen Flüssigkeitsleitung (6) ist, **dadurch gekennzeichnet, dass** die gemeinsame Flüssigkeitsleitung (6) eine Schlauchleitung ist, die unter Ausbildung der Verwirbelungselemente in einem vorgegebenen Abschnitt (6') der Schlauchleitung (6) verformt ist, und
dass die Mittel (10) zum Mischen der Blutkomponente und der Additivlösung eine Vorrichtung (20) zum Einlegen der Schlauchleitung (6) aufweisen, wobei die Vorrichtung an der Schlauchleitung angreifende Verformungselemente (21A, 2 1 B) aufweist.

2. Vorrichtung nach Anspruch 1, **dadurch gekennzeichnet, dass** zur Ausbildung der Verwirbelungselemente die von der Blutkomponente und der Additivlösung durchströmte Schlauchleitung (6) in vorgegebenen Abständen (a) derart verformt ist, dass der Abstand zwischen zwei auf einer ersten Achse gegenüberliegenden Punkten (P1, P2) der Schlauchleitung geringer ist als der Abstand zwischen zwei gegenüberliegenden Punkten (P3, P4), die auf einer zweiten im rechten Winkel zu der ersten Achse verlaufenden Achse liegen.

3. Vorrichtung nach Anspruch 2, **dadurch gekennzeichnet, dass** zur Ausbildung der Verwirbelungselemente die von der Blutkomponente und der Additivlösung durchströmte Schlauchleitung (6) in vorgegebenen Abständen (a) Strömungsquerschnitte in Form von im wesentlichen ovalen Flächen aufweist, wobei die Hauptachsen (A) der ovalen Flächen in unterschiedliche Richtungen verlaufen.

4. Vorrichtung nach einem der Ansprüche 1 bis 3, **dadurch gekennzeichnet, dass** die Vorrichtung zum Einlegen der Schlauchleitung ein Kunststoff-Clip ist.

5. Verfahren zum Filtrieren von Blut, bei dem eine Blutkomponente und eine Additivlösung für die Blutkomponente miteinander gemischt und die Mischung aus Blutkomponente und Additivlösung gefiltert werden, wobei zum Vermischen der Blutkomponente und der Additivlösung die Mischung von Blutkomponente und Additivlösung vor dem Filtrieren verwirbelt wird, **dadurch gekennzeichnet, dass** die Mischung aus Blutkomponente und Additivlösung durch eine Schlauchleitung (6) geleitet wird, die in vorgegebenen Abständen (a) unterschiedlich verformt ist, wobei die Schlauchleitung (6) in eine Vorrichtung eingelegt wird, die derart ausgebildet ist, dass die Schlauchleitung in vorgegebenen Abständen (a) verformt wird.

6. Verfahren nach Anspruch 5, **dadurch gekennzeichnet, dass** die Schlauchleitung (6) in vorgegebenen Abständen derart verformt ist, dass der Abstand zwischen zwei auf einer ersten Achse gegenüberliegenden Punkten (P1, P2) der Schlauchleitung geringer ist als der Abstand (P3, P4) zwischen zwei gegenüberliegenden Punkten, die auf einer zweiten im rechten Winkel zu der ersten Achse verlaufenden Achse liegen.

7. Verfahren nach Anspruch 6, **dadurch gekennzeichnet, dass** die Schlauchleitung (6) in vorgegebenen Abständen Strömungsquerschnitte in Form von im Wesentlichen ovalen Flächen aufweist, wobei die Hauptachsen (A) der ovalen Flächen in unterschiedliche Richtungen verlaufen.

8. Verfahren nach einem der Ansprüche 5 bis 7, **dadurch gekennzeichnet, dass** die Blutkomponente und/oder Additivlösung mit einer Pumpe (12') gefördert wird.

9. Verfahren nach einem der Ansprüche 5 bis 8, **dadurch gekennzeichnet, dass** die Schlauchleitung (6) in einen Kunststoff-Clip eingelegt wird, der die Schlauchleitung in vorgegebenen Abständen (a) verformt.

## Claims

1. A device for filtering a blood component and an additive solution with
a first chamber (1) for receiving a blood component,
a second chamber (2) for receiving an additive solution for the blood component,
means (10) for mixing the blood component and the additive solution, which comprise a body through which the blood component and the additive solution flow, the flow cross-section of said body being constituted differently for the formation of swirling elements generating swirls at predetermined intervals (a),
a first fluid line (3), which leads from the first chamber to the means for mixing the blood component and the additive solution and
a second fluid line (4), which leads from the second chamber to the means for mixing the blood component and the additive solution, and
a third fluid line (6), which leads from the means (10) for mixing the blood component and the additive solution to means (7) for filtering the mixture of blood component and additive solution,
wherein
the first fluid line (3) and the second fluid line (4) lead to a common fluid line (6), and the body through which the blood component and the additive solution flow is part of a common fluid line (6), **characterised in that** the common fluid line (6) is a hose line, which is deformed thereby forming the swirling elements in a predetermined section (6') of the hose line (6), and
that the means (10) for mixing the blood component and the additive solution comprise a device (20) for inserting the hose line (6), wherein the device comprises deformation elements (21A, 21B) acting on the hose line.

2. The device according to claim 1, **characterised in that**, for the formation of the swirling elements, the hose line (6) through which the blood component and the additive solution flow is deformed at predetermined intervals (a) in such a way that the interval between two points (P1, P2) of the hose line lying opposite on a first axis is smaller than the interval between two points (P3, P4) lying opposite which lie on a second axis running at right angles to the first axis.

3. The device according to claim 2, **characterised in that**, for the formation of the swirling elements, the hose line (6) through which the blood component and the additive solution flow comprises flow cross-sections in the form of essentially oval areas at predetermined intervals (a), wherein the principal axes (A) of the oval areas run in different directions.

4. The device according to any one of claims 1 to 3, **characterised in that** the device for inserting the hose line is a plastic clip.

5. A method for filtering blood, wherein a blood component and an additive solution for the blood component are mixed together and the mixture comprising blood component and additive solution are filtered, wherein the mixture of blood component and additive solution is swirled before the filtering in order to mix the blood component and the additive solution, **characterised in that** the mixture comprising blood component and additive solution is conveyed through a hose line (6), which is deformed differently at predetermined intervals (a), wherein the hose line (6) is inserted into a device which is constituted such that the hose line is deformed at predetermined intervals (a).

6. The method according to claim 5, **characterised in that** the hose line (6) is deformed at predetermined intervals, in such a way that the interval between two points (P1, P2) of the hose line lying opposite on a first axis is smaller than the interval (P3, P4) between two points lying opposite which lie on a second axis running at right angles to the first axis.

7. The method according to claim 6, **characterised in that** the hose line (6) comprises flow cross-sections in the form of essentially oval areas at predetermined intervals, the principal axes (A) of the oval areas running in different directions.

8. The method according to any one of claims 5 to 7, **characterised in that** the blood component and/or additive solution is conveyed with a pump (12').

9. The method according to any one of claims 5 to 8, **characterised in that** the hose line (6) is inserted into a plastic clip, which deforms the hose line at predetermined intervals (a).

## Revendications

1. Dispositif de filtration d'un constituant sanguin et d'une solution additive avec
(i) une première chambre (1) destinée à recevoir un constituant sanguin,
(ii) une deuxième chambre (2) destinée à recevoir une solution additive pour le constituant sanguin,
(iii) des moyens (10) pour mélanger le consti-tuant sanguin et la solution additive, qui présentent un corps traversé par le consti-tuant sanguin et la solution additive, dont la section mouillée est configurée de manière différente à des distances prédé-terminées (a) en vue de former des éléments de turbulence produisant des turbulences,
(iv) une première conduite de liquide (3), qui conduit de la première chambre aux moyens pour mélanger le constituant sanguin et la solution additive, et
(v) une deuxième conduite de liquide (4), qui conduit de la deuxième chambre aux moyens pour mélanger le constituant sanguin et la solution additive, et
(vi) une troisième conduite de liquide (6), qui conduit des moyens (10) pour mélanger le constituant sanguin et la solution additive à des moyens (7) pour filtrer le mélange de constituant sanguin et de solution additive,
dans lequel la première conduite de liquide (3) et la deuxième conduite de liquide (4) condui-sent à une conduite de liquide commune (6), et le corps traversé par le constituant sanguin et la solution additive fait partie de la conduite de liquide commune (6), **caractérisé en ce que** la conduite de liquide commune (6) est une conduite souple, qui est déformée dans une partie prédéterminée (6') de la conduite souple pour former les éléments de turbulence, et **en ce que** les moyens (10) pour mélanger le constituant sanguin et la solution additive présentent un dispositif (20) pour insérer la conduite souple (6), dans lequel le dispositif présente des éléments de déformation (21A, 21B) agissant sur la conduite souple.

2. Dispositif selon la revendication 1, carac-térisé en ce que, pour former les éléments de turbulence, la conduite souple (6) traversée par le constituant sanguin et la solution additive est déformée à des distances prédéterminées (a), de telle manière que la distance entre deux points opposés (P1, P2) de la conduite souple sur un premier axe soit plus petite que la distance entre deux points opposés (P3, P4), qui sont situés sur un deuxième axe orienté perpendiculairement au premier axe.

3. Dispositif selon la revendication 2, carac-térisé en ce que, pour former les éléments de turbulence, la conduite souple (6) traversée par le constituant sanguin et la solution additive présente, à des distances prédéterminées (a), des sections mouillées en forme de surfaces essentiellement ovales, dans lequel les axes principaux (A) des surfaces ovales s'étendent dans des directions différentes.

4. Dispositif selon l'une quelconque des revendications 1 à 3, **caractérisé en ce que** le dispositif pour insérer la conduite souple est un clip en matière plastique.

5. Procédé de filtration de sang, dans lequel on mélange l'un avec l'autre un constituant sanguin et une solution additive pour le constituant sanguin et on filtre le mélange du constituant sanguin avec la solution additive, dans lequel on soumet le mélange du constituant sanguin et de la solution additive à des turbulences afin de mélanger intimement le constituant sanguin et la solution additive avant la filtration, **caractérisé en ce que** l'on conduit le mélange du constituant sanguin et de la solution additive à travers une conduite souple (6), qui est défor-mée de façon différente à des distances prédé-terminées (a), dans lequel on insère la conduite souple (6) dans un dispositif qui est configuré de telle manière que la conduite souple soit déformée à des distances prédéterminées (a).

6. Procédé selon la revendication 5, **caractérisé en ce que** la conduite souple (6) est déformée à des distances prédéterminées (a), de telle manière que la distance entre deux points opposés (P1, P2) de la conduite souple sur un premier axe soit plus petite que la distance entre deux points opposés (P3, P4), qui sont situés sur un deuxième axe orienté perpendicu-lairement au premier axe.

7. Procédé selon la revendication 6, **caractérisé en ce que** la conduite souple (6) présente à des distances prédéterminées des sections mouillées en forme de surfaces essentiellement ovales, dans lequel les axes principaux (A) des surfaces ovales s'étendent dans des directions diffé-rentes.

8. Procédé selon l'une quelconque des revendica-tions 5 à 7, **caractérisé en ce que** le composant sanguin et/ou la solution additive est fourni(e) par une pompe (12')_{.}

9. Procédé selon l'une quelconque des revendica-tions 5 à 8, **caractérisé en ce que** l'on insère la conduite souple (6) dans un clip en matière plastique, qui déforme la conduite souple à des distances prédéterminées (a).
